# EUROPEAN PATENT SPECIFICATION

(11) **EP 3 113 721 B1**
(45) Date of publication and mention of the grant of the patent: **06.06.2018**
(21) Application number: 15713654.0
(22) Date of filing: 09.03.2015
(51) Int. Cl.: A61F 2/06

(54) **CATHETER DEVICE FOR FENESTRATING A STENTGRAFT**
KATHETERVORRICHTUNG ZUR FENESTRIERUNG EINES STENTGRAFTS
DISPOSITIF DE CATHÉTER SERVANT À RÉALISER UNE FENESTRATION D'UNE ENDOPROTHÈSE

(30) Priority: 07.03.2014 EP 14158326
(43) Date of publication of application: 11.01.2017
(73) Proprietor: Maquet Holding B.V.&Co. KG, 76437 Rastatt (DE); Berg, Patrick, 40670 Meerbusch (DE); Berg, Victor, 40670 Meerbusch (DE)
(72) Inventor: Berg, Patrick, 40670 Meerbusch (DE); Berg, Victor, 40670 Meerbusch (DE)
(74) Representative: Schaumburg und Partner Patentanwälte mbB
(86) International application number: PCT/EP2015/054854
(87) International publication number: WO 2015/132417

(56) References cited:
- EP-A1- 0 941 698
- WO-A1-94/13211
- WO-A1-2009/056644
- US-A1- 2010 106 175
- US-A1- 2012 041 544

## Description

### FIELD OF THE INVENTION

The present invention relates generally to devices usable to create puncture holes into the fabric of a stentgraft within the body of a patient.

### BACKGROUND OF THE INVENTION

In the following we refer to a list of references at the end of the description.

An aortic aneurysm is characterized by the dilatation of the wall of the aorta. Aortic aneurysms are likely to rupture, especially when the diameter increases, thus posing significant danger to the patient. Rupture of an aneurysm gives only a 20 % chance of survival, so there is a significant emphasis placed on early diagnosis and treatment.

Aneurysms of the abdominal and thoracic aorta are a major cause of mortality, especially in the western societies. With an increasingly ageing society, the incidence of aneurysm, particular abdominal aortic aneurysm, continues to rise.

Surgical repair of aneurysm is a major and invasive undertaking and there has been much effort in developing less invasive methods. Endovascular repair of aneurysms, using stentgrafts, is now accepted the method of choice. Stentgrafts are medical devices constructed to reinforce, replace, or bridge a part of a damaged, unhealthy, or diseased blood vessel. The technique has significant limitations and is nor suitable for all patients.

The difficulty of using a stentgraft at or near an intersection of an artery to be repaired (such as the aorta) with branch vessels (for example the renal arteries, superior mesenteric artery, celiac trunk, brachiocephalic artery, carotid arteries or left subclavian artery etc.) is well recognized. For such patients, endovascular repair is only possible using unique, individually designed and manufactured endografts having appropriate branch grafts or fenestrations, which match the patient's anatomy. Whilst such grafts can be obtained commercially, for example the Zenith device of COOK or the fenestrated Anaconda graft from VASCUTEK, the meticulous design of such grafts is reliant on accurate preoperative imaging data.

The use of patient-specific designed grafts is expensive and requires significant pre-planning so that such grafts are not available in emergency situations.

Proof of concept for off-the-shelf fenestrated devices (VENTANA fenestrated system, COOK Zenith p-branch) for the endovascular repair of juxtarenal and pararenal aortic aneurysms in selected patients has been demonstrated. But nearly 40% of juxtarenal or pararenal aneurysms do not meet anatomical criteria for endovascular repair using one of the two devices, justifying need for additional designs (Mendes et al., 2013).

The desirability of conducting in vivo (in situ) fenestration of a graft has been recognized (McWilliams et al., 2004). McWilliams et al. describe the fenestration of a thoracic graft by passing a guide wire down the branch artery to pierce the fabric of the graft and then expanding the hole formed by inflation of a balloon. However, whilst the technique described shows that in situ fenestration is possible, it requires percutaneous retrograde access to the branch vessel for correct location of the fenestration. Such is possible for branches of the aortic arch (Manning et al., 2010)(McWilliams et al., 2004) but not for visceral vessels such as the renal or mesenteric arteries. Retrograde Fenestration for visceral vessels has been described before (McWilliams et al., 2003) but only in animal experiments.

An alternative approach is to puncture the graft fabric in an antegrade fashion. Such a technique has been described using radiofrequency based apparatus (Biadillah et al., 2012), needle puncture of bloused section (Bruszewski et al., 2009a; Bruszewski et al., 2009b; Bruszewski et al., 2009b), magnetic location and needle puncture (Berg, 2010), needle puncture (Mafi and Bruszewski, 2009), piercing catheter (McLachlan et al., 2010), stent (Naor and Ackerman, 2012), steerable catheter and puncture (Wallace and Stahler, 2009), temporary "chimney" (Wolf, 2012) or sidebranch stentgraft (Shaw, 2012).

Document EP 0 941 698 A1 discloses a suturing device allowing a physician to remotely seal an incision in a blood vessel or other biological tissue. The device comprises an elongated tubular body having a distal portion which is adapted to be inserted percutaneously through the incision and into the blood vessel. The distal portion has at least first and second retractable arms which extend from the distal portion of the body and releasably hold a suture within the blood vessel. First and second retractable needles, each of which is configured to catch the suture from a respective arm, are provided along the body proximal to the retractable arms. The arms and the needles are remotely movable by the physician using a handle or other control mechanism provided at a distal portion of the device. In operation, the arms are initially deployed within the blood vessel to hold the ends of the suture beyond the circumference of the tubular body. The needles are then deployed from and then retracted into the body during which time the needles pierce the wall of the vessel on substantially opposite sides of the incision, release the suture ends form the retractable arms, and pull the suture through the vessel wall. The device is particularly useful for closing an incision in an artery following a catheterization procedure.

It is the object of the invention to provide a catheter device enabling safe and easy fenestration of stentgrafts that have not been adapted to the individual patient.

### SUMMARY OF THE INVENTION

According to the invention, the catheter device for fenestrating a stentgraft comprises a first needle catheter with a needle having a barbed hook and a thread which is connected to the needle. Thereby the catheter device allows for a retrograde fenestration of the stentgraft, wherein the stentgraft can be pierced by the needle. The barbed hook prevents a retraction of the needle, and the thread can be fed through an orifice that has been created by the needle in the stentgraft when the needle is moved through the orifice.

The needle can be placed inside of any branch vessel with the tip of the needle pointing towards the opening of the branch vessel to a blood vessel. The needle can come out of the branch vessel, which is also referred to as target vessel, and perforate the stentgraft at the right place just in front of the ostium of the target vessel. The direction of the needle is pointed at the center of the aorta, whereby the risk of an accidental puncture of body tissue is reduced. Preferable the material and shape of the needle are adapted to penetrate the stentgraft which preferably consists at least partly of fabric. Further, a preferable material for the thread is nylon.

Preferably, the catheter device further comprises a balloon catheter with a balloon that is piercable by the needle such that the barbed hook prevents a retraction of the needle from the balloon. It is thus possible to capture the needle from a side of the stentgraft opposite to the side from which the needle has pierced the stent-graft. Further, the simultaneous inflating of the balloon of the balloon catheter prevents an accidental puncture of the aorta.

In a preferable embodiment the catheter device further comprises a second needle catheter with a hollow needle through which the thread is feedable. Thereby the orifice that has been created by piercing the needle through a fabric of the stent-graft can be further enlarged. The second needle catheter enlarges the orifice or puncture hole also in a retrograde manner from the outside of the fabric to the inner side of the fabric. The nylon thread guides the direction, whereby the hollow needle can be precisely positioned at the location of the orifice and being pierced though the fabric of the stentgraft. Preferably, the diameter of the hollow needle is bigger than the diameter of the needle.

Preferably, the catheter device further comprises an introducer sheath into which the hollow needle is feedable in such a manner that an accidental puncture is prevented. It is thus achieved that the hollow needle only pierces the fabric of the stentgraft at the intended location. After perforating the fabric, the needle enters the introducer sheath on the other side preventing an accidental puncture.

Preferably, the first needle catheter of the catheter device comprises an appliance for moving the needle in a specified direction. It is thus possible to pierce the fabric of the stentgraft at a suitable location, i.e. in front of the ostium of the branch vessel.

In a further advantageous embodiment, the first needle catheter of the catheter device has a bendable tip. Further, the first needle catheter comprises a steering catheter and the appliance is connected to a bendable tip of the steering catheter that is located in the bendable tip of the first needle catheter. These features enable the tip of the first needle catheter to be bent from the blood vessel, in which the major part of the first needle catheter is located, into the branch vessel. Thus, the location where the fabric of the stentgraft should be fenestrated can easily be determined without any risk of harming the patient.

In a further preferred embodiment of the catheter device, the appliance comprises an elongated guiding element and a shiftable element that is slidably supported by the guiding element in such a manner that the shiftable element is shiftable along a longitudinal axis of the guiding element. Further, the needle is movable by shifting the shiftable element in the specified direction. Thus, the appliance for moving the needle in the specified direction can be implemented by a simple structure.

Preferably, the guiding element of the catheter device is a hollow cylinder, and the shiftable element is located inside of the hollow cylinder. Thus, the appliance is resistant to outer influences, and that the needle can be moved reliably in the specified direction which is parallel to the longitudinal axis of the hollow cylinder.

In another advantageous embodiment of the catheter device, the shiftable element comprises a pressure pin and a further hollow cylinder for supporting the needle. Further, the needle is locatable in such a manner that the needle extends into the further hollow cylinder and that an end of the needle opposite to the tip of the needle abuts against the striker pin. Thus, it is ensured that the tip of the needle is moved in the specified direction when the shiftable element is shifted.

Preferably, a further thread is connected to the shiftable element in a connection area thereof. The further thread is fed through an orifice of the guiding element that is located at a distance from the connection area in the specified direction. Thus, the shiftable element can easily be moved by pulling the further thread through the orifice.
It is preferable that the specified direction is a direction opposite to the direction in which the tip of the first needle catheter points. Thus, the tip of the first needle catheter can be bent into the branch vessel such that the tip of the needle points automatically in a direction suitable for piercing the fabric of the stentgraft.
Preferably, the balloon of the catheter device has an inner layer and an outer layer, wherein the inner layer is thicker than the outer layer. Thus, the balloon keeps its shape when the needle pierces the balloon, in particular to prevent a bursting of the balloon, and to ensure the stability of the balloon. Preferably, both layers consist of latex or a material having corresponding mechanical properties.
In a method for fenestrating a stentgraft located in a blood vessel, which branches into a branch vessel, a first needle catheter, having a needle catheter adapated to fenestrate the stentgraft, is introduced in a retrograde manner into the blood vessel, the needle comprising a barbed hook and being connected to a thread. The method can be applied to a large variety of blood vessels and branch vessels, in particular the fenestration can be performed in a safe manner without any risk of accidently puncturing body tissue of the patient.

In a described method, a tip of the first needle catheter is bent and introduced into the branch vessel. Thus, the location for the fenestration of the stentgraft can easily be determined.

The method can further be improved, if, as a next step, the stentgraft is introduced into the blood vessel in a region in which the blood vessel branches into the branch vessel in such a manner that an outer side of a fabric of the stentgraft faces the tip of the first needle catheter. By performing this step after bending the tip of the first needle catheter and introducing the tip of the first needle catheter into the branch vessel, it is easy to locate the outer side of the fabric of the stentgraft such that it faces the first needle catheter.

Preferably, a balloon catheter with an inflatable balloon is introduced through an introducer sheath in an antegrade manner. The balloon is located on an inner side of the fabric of the stentgraft which is opposed to the outer side of the fabric. Subsequently, the balloon is inflated. By these steps it can be ensured that the needle is connected to the balloon when the needle pierces though the fabric of the stent-graft and the risk of accidentally puncturing the body tissue can be reduced.

The method can further be improved by piercing the needle through the fabric of the stentgraft from the outer side thereof so that an orifice is created in the fabric of the stentgraft through which the thread is fed, by piercing the needle through a surface of the balloon and locating the barbed hook inside of the balloon and by removing the balloon and the first needle catheter. Thus, it is achieved that the thread is fed through the orifice and can be used as a guide wire for other instruments that operate on the fabric in the area of the orifice.

Preferably, the thread is tensioned and a second needle catheter with a hollow needle through which the thread is fed is moved in a retrograde manner towards the outer side of the fabric of the stentgraft, wherein the hollow needle has a diameter larger than the diameter of the needle. Further, the tip of hollow needle is pierced through the fabric of the stentgraft along the thread at a location where the orifice of the stentgraft is located. Thereafter the second needle catheter is removed. Thereby the orifice is enlarged.

Preferably, before the tip of the hollow needle is pierced through the fabric of the stentgraft, the introducer sheath is moved towards the fabric of the stentgraft until a first end of the introducer sheath faces the inner side of the fabric of the stent-graft. Thus, it is ensured that the hollow needle does not accidently puncture the stentgraft in unintended locations or body tissues of the patient.

Preferably, an enlargement balloon is introduced in an antegrade manner along the thread. The enlargement balloon is fed through the orifice until the orifice is enlarged. Then, the enlargement balloon is removed. Thus, the orifice is further enlarged in a simple and safe manner.

Further steps comprise introducing a diagnostic catheter with an angled tip through the introducer sheath in an antegrade manner, moving the angled tip of the diagnostic catheter through the orifice of the fabric of the stentgraft, feeding a flexible guide wire through the orifice and through a part of the branch vessel and removing the diagnostic catheter. Thus, it is ensured that further instruments can be guided along the guide wire to establish a channel between the stentgraft and the branch vessel.

Preferably, the flexible guide wire is replaced by a stiff wire. Further, an introducer balloon is introduced over the stiff wire in an antegrade manner until a first part thereof is located on the outer side of the fabric and a second part thereof is located on the inner side of the fabric. At the same time the introducer sheath is pushed towards the orifice of the fabric and the introducer balloon is subsequently inflated and deflated until the introducer sheath enters through the orifice of the fabric along the stiff guide wire into the branch vessel. Thereafter, the introducer balloon is removed. Thus, it is ensured that the introducer sheath enters the branch vessel and lies against the rim of the orifice.

Further advantageous steps include introducing a covered side branch stent over the stiff wire until it is fed through the orifice and extends into the branch vessel. Thus, a permanent channel from the blood vessel into the branch vessel is established.

Preferably, a further thread that is connected to an appliance for moving the needle in a specified direction is pulled for moving the needle through the fabric of the stentgraft. Thus, the needle can easily be moved in a reliable and controllable manner.

### BRIEF DESCRIPTION OF THE DRAWINGS

Further features and advantages of the invention can be derived from the following description which explains the invention in more detail on the basis of embodiments in connection with the enclosed Figures, wherein:
- Figure 1: shows a perspective view of a first introducer sheath and a guide wire;
- Figure 2: shows a perspective view of the first introducer sheath and a first needle catheter;
- Figure 3: shows a perspective view of the first introducer sheath and the first needle catheter, wherein the first introducer sheath and parts of the first needle catheter are shown transparently for illustrational purposes;
- Figure 4: shows a sectional view along the line A-A indicated in Figure 2;
- Figure 5: shows elements of an appliance of the first needle catheter;
- Figure 6: shows a perspective view of a balloon catheter;
- Figure 7: shows the balloon catheter and a needle that penetrates the surface of a balloon of the balloon catheter;
- Figure 8: shows a perspective view of a second introducer sheath, a second needle catheter and a third introducer sheath;
- Figure 9: shows a sectional view of the first introducer sheath which is introduced into the aorta of a patient in the region of an aneurysm;
- Figure 10: shows a sectional view of a stentgraft, the first introducer sheath and the first needle catheter in a branch region of the branch artery and the aorta above the aneurysm;
- Figure 11: shows a sectional view of the branch region of the branch artery and the aorta, wherein the balloon catheter has been introduced through the third introducer sheath into the aorta;
- Figure 12: shows a sectional view of the branch region of the branch artery and the aorta, wherein the balloon of the balloon catheter has been inflated;
- Figure 13: shows a sectional view of the branch region of the branch artery and the aorta after the balloon catheter and the first introducer sheath have been removed together with the first needle catheter;
- Figure 14: shows a sectional view of the branch region of the branch artery and the aorta, wherein a second introducer sheath and a third introducer sheath have been introduced into the aorta;
- Figure 15: shows a sectional view of the branch region of the branch artery and the aorta, wherein a tip of the second needle catheter is moved close to an orifice of the fabric of the stentgraft;
- Figure 16: shows a sectional view of the branch region of the branch artery and the aorta, wherein the second introducer sheath and the second needle catheter have been removed and an angioplasty balloon catheter has been introduced;
- Figure 17: shows a sectional view of the branch region of the branch artery and the aorta, wherein the angioplasty balloon catheter has been removed and a diagnostic catheter has been introduced;
- Figure 18: shows a sectional view of the branch region of the branch artery and the aorta, wherein a PTA balloon has been introduced after the diagnostic catheter has been removed;
- Figure 19: shows a sectional view of the branch region of the branch artery and the aorta after the third introducer sheath has entered the branch vessel;
- Figure 20: shows a sectional view of the branch region of the branch artery and the aorta, wherein a side branch stent has been placed into the branch artery to seal the stentgraft.

### DETAILED DESCRIPTION OF THE INVENTION

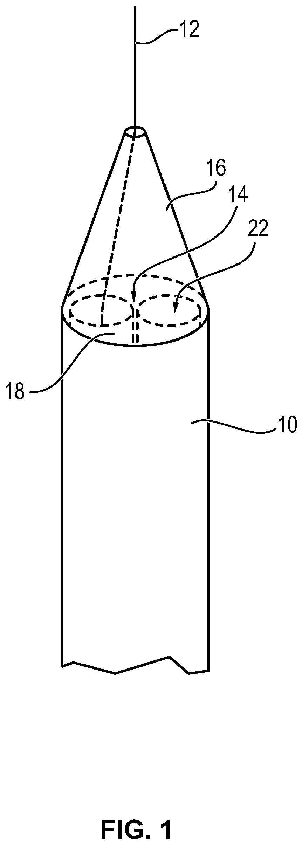

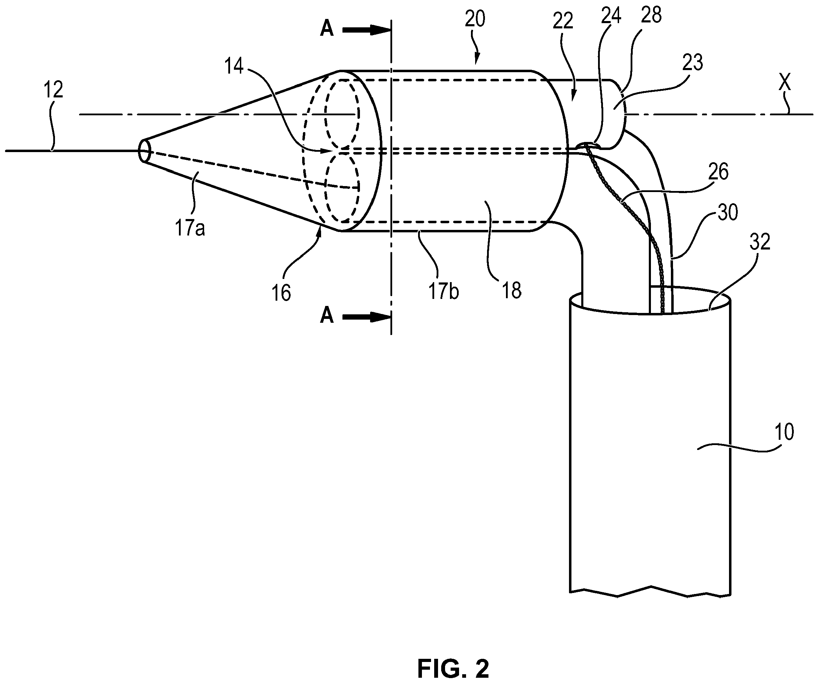

Figures 1 and 2 show a perspective view, respectively, of a first introducer sheath 10 that glides over a guide wire 12 with a diameter of e.g. 0.035". The first introducer sheath 10 contains a first needle catheter 14 with its tip acting as a dilator 16.

The first needle catheter 14 is made of a steerable catheter 18 with a tip 20 to be bent to 90 degrees and a mounted appliance 22 fixed on the bending part. While pulling the first introducer sheath 10 slightly back, the bending part 20 of the first needle catheter 14 and the appliance 22 together with the dilator 16 are visible.

The dilator 16 has a conical part 17a and a cylindrical part 17b which enfolds a part of the steerable catheter 18 and a part of the appliance 22 so that they are connected. The appliance 22 comprises a hollow cylinder 23 with a longitudinal axis X and an orifice 24 through which thread 26 is fed. The hollow cylinder 23 has an opening 28 through which a further thread 30 is fed. The proximal parts of the threads 26 and 30 are both fed, respectively, through an opening 32 of the first introducer sheath 10. Preferably, the threads 26 and 30 consist of nylon.

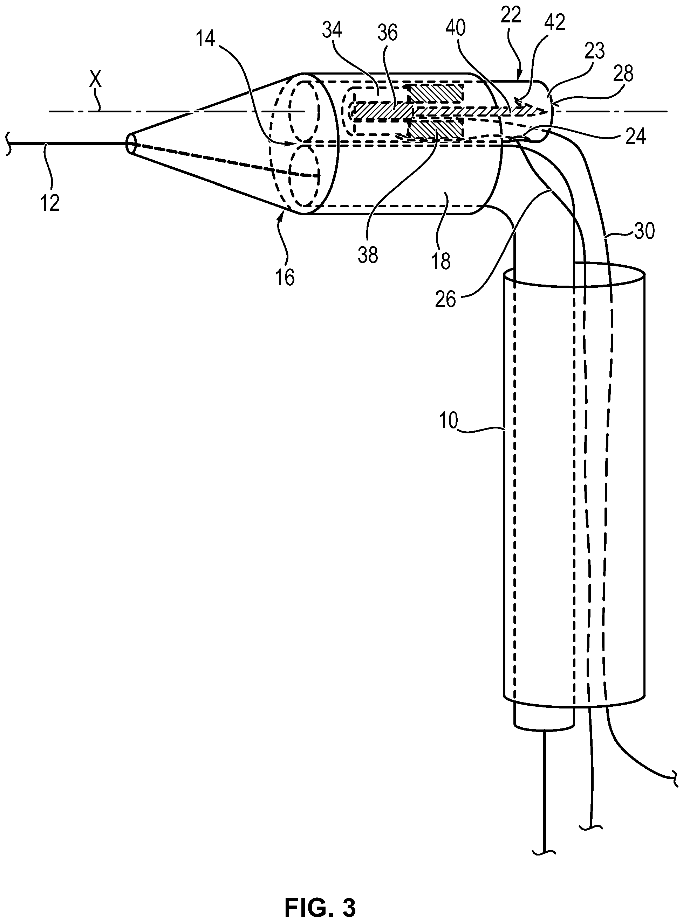

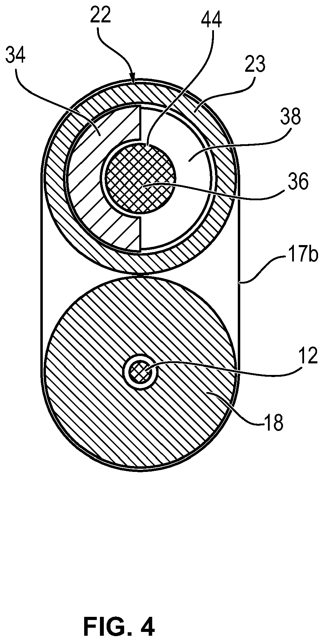

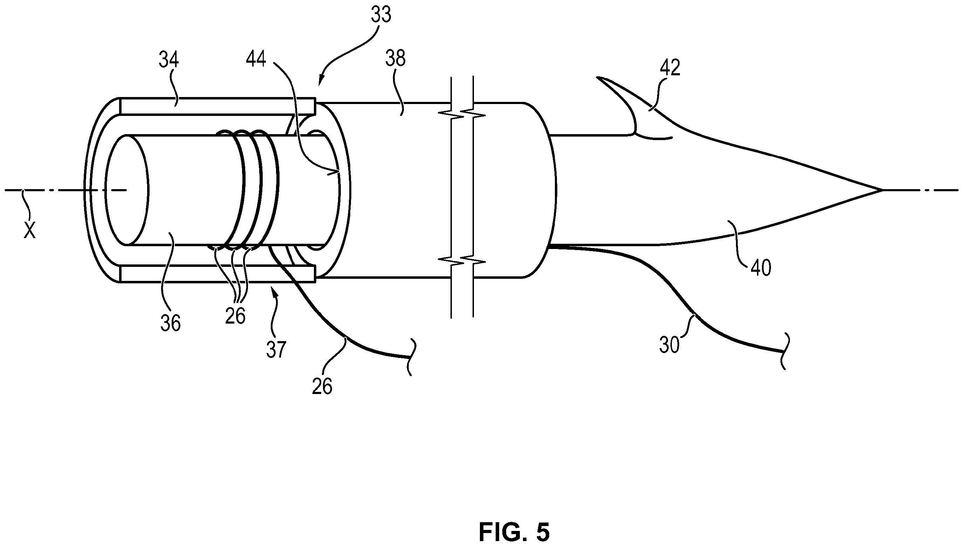

Figure 3 shows a perspective view of the first introducer sheath 10 and the first needle catheter 14, wherein the first introducer sheath 10, the appliance 22 and the dilator 16 of the first needle catheter 14 are shown transparently for illustrational purposes and elements that are located inside of the cylinder 23 are shown in a sectional view. Figure 4 shows a sectional view along the line A-A indicated in Figure 2, in which details of the appliance 22 are shown. Figure 5 shows the elements of the appliance 22 that are contained inside of the hollow cylinder 23.

The appliance 22 contains a half cylinder 34 fixed on the nylon thread 26. A piece of metal 36 is fixed on the thread 26 and on a smaller cylinder 38, where a needle 40 with a barbed hook 42 is placed. The area in which the thread 26 is connected to the piece of metal 36 is denoted by 37 in Figure 5.

Further, the half cylinder 34 is slidably supported by the hollow cylinder 23. The half cylinder 34, the piece of metal 36 and the cylinder 38 form a shiftable element 33 that is shiftable along the longitudinal axis X. In Figure 3 only a part of the cylinder 38 is shown so that the needle 40 is visible.

As shown in Figure 4, the cylinder 38 has a concentric through hole in which a back end of the needle 40 opposite to the tip thereof is located. The back end of the needle 40 abuts against the piece of metal 36 which acts as a pressure pin that pushes the needle 40 along the longitudinal axis X towards the opening 28 when the thread 26 is pulled through the orifice 26 out of the hollow cylinder 23.

The needle 40 with the barbed hook 42 is also connected with the nylon thread 30, which is fed through the opening 28 of the hollow cylinder 23.

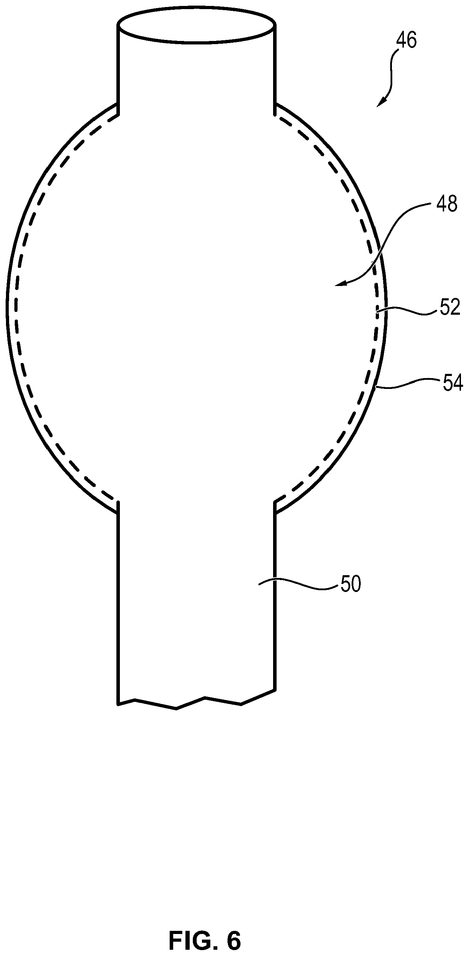

Figure 6 shows a perspective view of a balloon catheter 46 with a balloon 48 and a catheter 50. The modelling balloon 48 is fixed on the catheter 50 which has two channels. One of the two channels is used to glide over a guide wire, e.g. a guide wire with a diameter of 0.035", the second channel is used to inflate the balloon 48. The balloon 48 is made of latex or a material having equivalent mechanical properties. It can be perforated without bursting of the balloon 48.

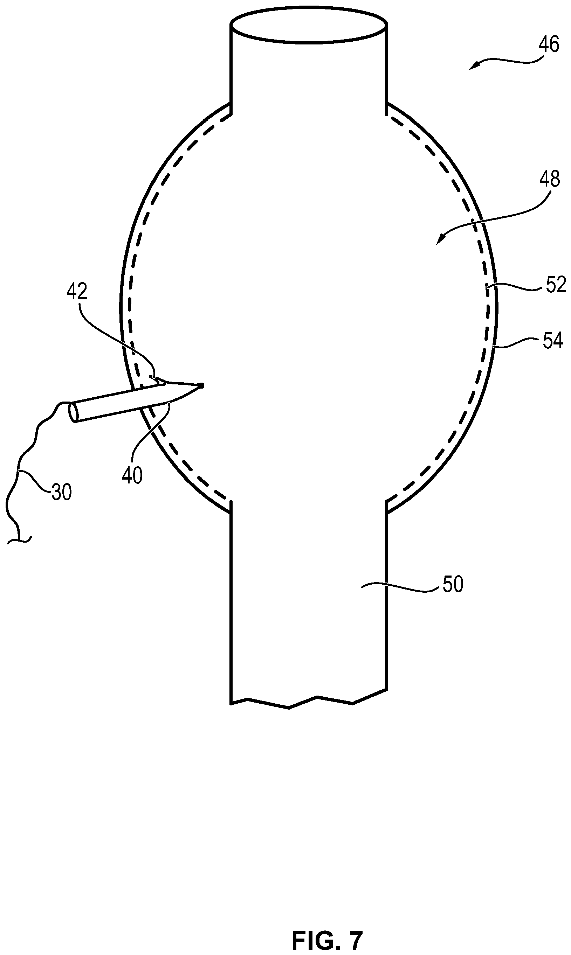

The balloon 48 contains two layers 52 and 54. The inner layer 52 is made of a thicker latex-like material and is meant to give stability in the vessel due to the greater thickness. The outer layer 54 is made of a thin latex-like material that is more stretchable and tear-resistant to catch the needle 40 with the barbed hook 42, as is shown in a perspective view in Figure 7.

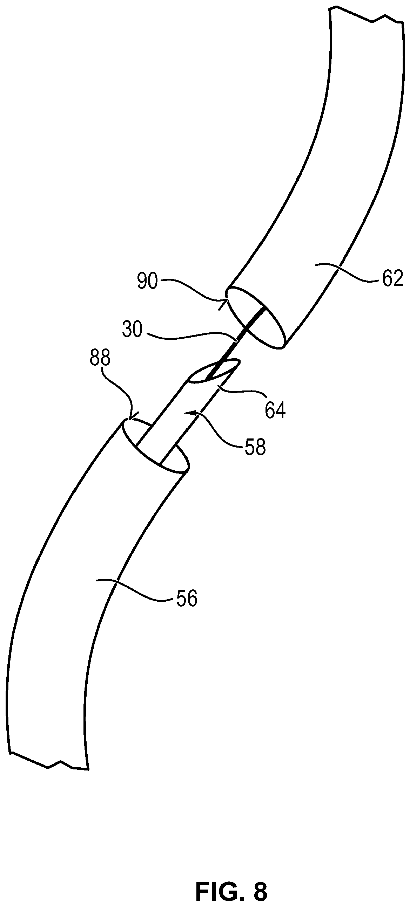

Figure 8 shows a perspective view of a second introducer sheath 56, a second needle catheter 58, the thread 30 and a third introducer sheath 62. The second introducer sheath 56 has an opening 88, and the third introducer sheath 62 has an opening 90. The openings 88 and 90 face each other. Further, the second needle catheter 58 is fed through the second introducer sheath 56 and has a hollow needle 64 at its tip that is located outside of the second introducer sheath 56. The hollow needle 64 and the second needle catheter 58 each have a channel through which the thread 30 is fed.

The following figures show a method to fenestrate a stentgraft 76 that is placed into the aorta 66 of a patient in order to channel the blood flow in the area of an aortic aneurysm 68.

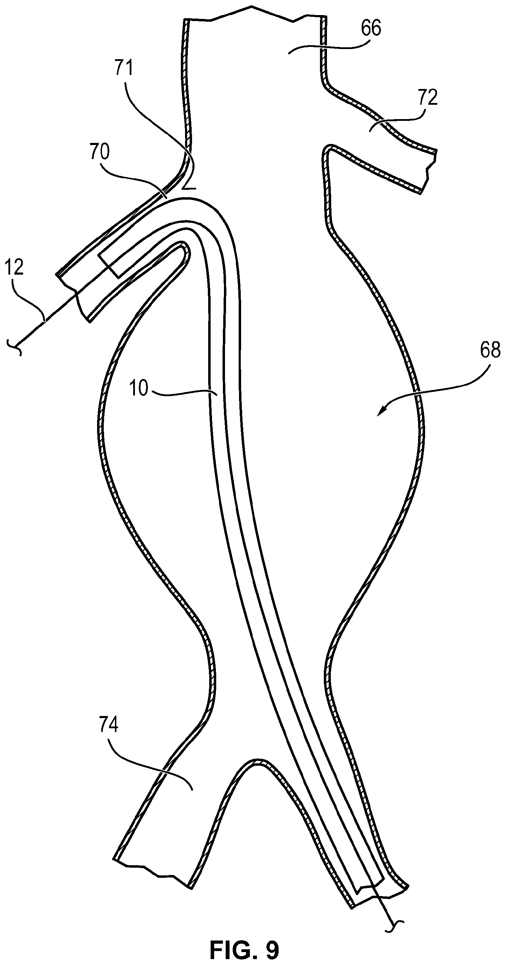

Figure 9 shows a sectional view of the first introducer sheath 10 which is introduced in a retrograde manner into the aorta 66 in the region of the aneurysm 68. Several branch arteries 70, 72 and 74 branch from the aorta 66 in the region of the aneurysm 68. The branch artery 70 having an opening 71 to the aorta 66 and the aorta 66 are cannulated in such a manner that the wire 12 is fed through a part of the branch artery 70 and a part of the aorta 66. The wire 12 is used to introduce the first introducer sheath 10 which is fed through the region of the aneurysm 68 and extends into the branch artery 70.

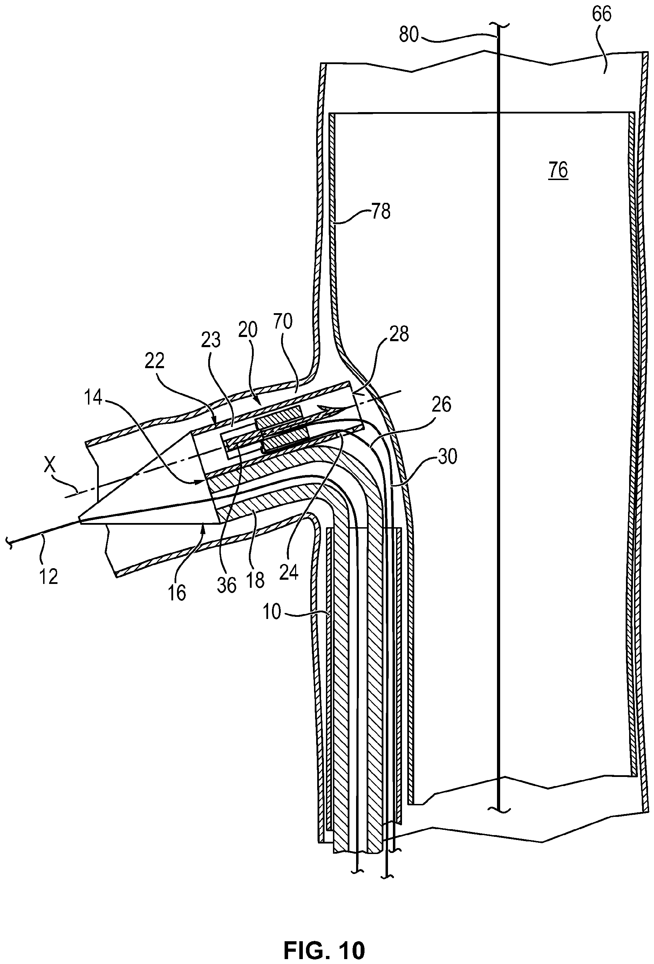

Figure 10 shows a sectional view of the stentgraft 76 and the first introducer sheath 10 in a branch region of the branch artery 70 and the aorta 66 above the aneurysm 68. The first introducer sheath 10 is moved backwards relative to its position in Figure 9 and relative to the first needle catheter 14 so that the appliance 22 is located outside of the introducer sheath 10. The tip 20 of the first needle catheter 14 is bent from the aorta 66 into the branch artery 70 so that the longitudinal axis X of the hollow cylinder 23 is parallel to the longitudinal axis of the branch artery 70 close to the branch region of the branch artery 70 and the aorta 66.

The opening 28 of the hollow cylinder 23 of the appliance 22 is located so close to a fabric 78 of the stentgraft 76 that the hollow cylinder 23 touches an outer side of the fabric 78. The stentgraft 76 has been introduced over a guide wire 80 which is fed through a part of the aorta 66.

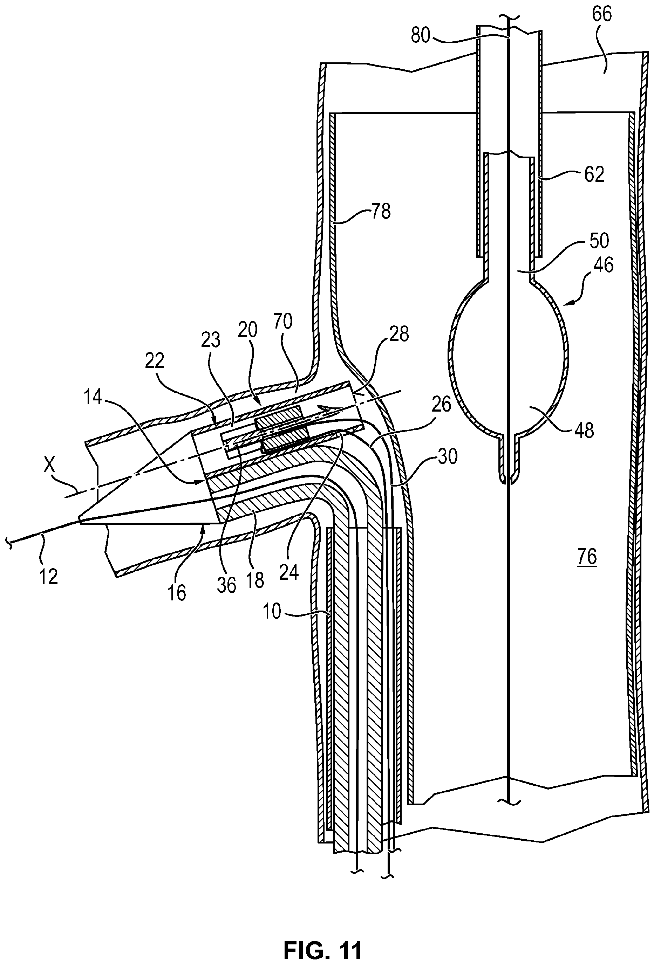

Figure 11 shows a sectional view of the region shown in Figure 10, wherein the balloon catheter 46 has been introduced through the third introducer sheath 62 into the aorta 66 over the guide wire 80 in an antegrade manner. The balloon 48 of the balloon catheter 46 is moved out of the third introducer sheath 62 and located in the branch region of the branch artery 70 and the aorta 66.

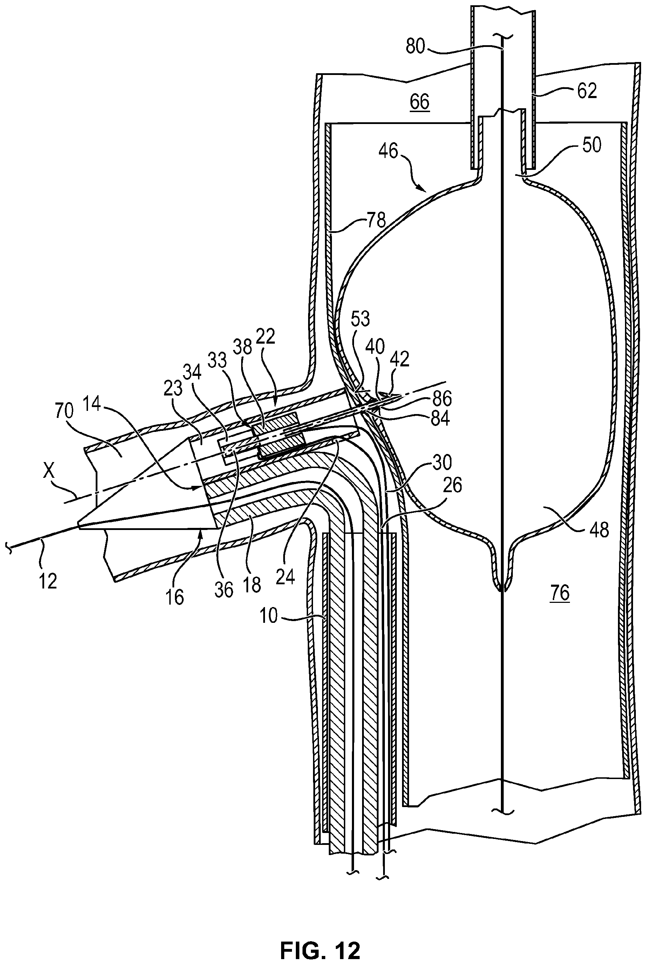

Figure 12 shows a sectional view of the branch region of the branch artery 70 and the aorta 66, wherein the balloon 48 has been inflated until the surface of the balloon 48 touches an area 53 of the fabric 78 of the stentgraft 76 on a inner side of the fabric 78 opposed to the opening 28 of the hollow cylinder 23 of the appliance 22.

The nylon thread 26 fixed on the cylindrical appliance 22 comes out of the appliance 22 through the small orifice 24 at the distal part of the appliance 22 on the lower side and enters the introducer sheath 10. By pulling on this nylon thread 26 the needle 40 is pushed out backwards to penetrate the fabric 78.

The simultaneous inflating of the balloon 48 of the balloon catheter 46 exerts a counter-pressure on the fabric 78 facilitating the perforation of the fabric 78. The needle 40 is pushed back through the fabric 78 of the stentgraft 76 into the fabric of the inflated balloon 48 so that an orifice 84 in the fabric 78 of the stentgraft 76 and an orifice 86 in the layers 52 and 54 of the balloon 48 are created. The double layer 52 and 54 of the balloon 48 captures the needle 40.

The barbed hook 42 of the needle 40 which has penetrated both layers 52 and 54 of the balloon 48 prevents the needle 40 from being pulled out of the balloon 48. While retrieving the balloon 48 the needle 40 with the attached wire is pulled back, so that the needle 40, the cylinder 38 and the end of the thread 30 that is attached to the needle 40 are moved through the orifice 84 in the fabric 78 of the stentgraft 76.

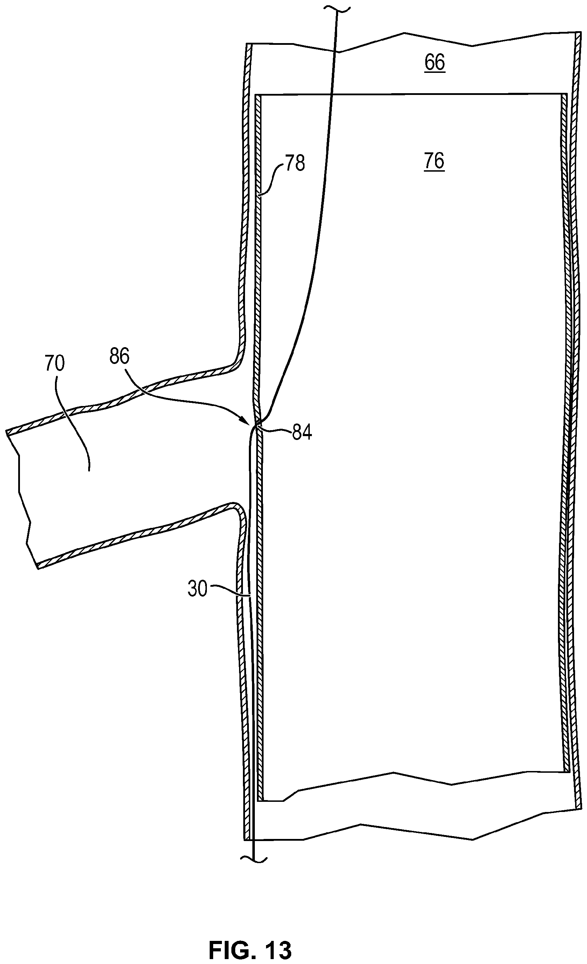

Figure 13 shows a sectional view of the branch region of the branch artery 70 and the aorta 66 after the balloon catheter 46 and the first introducer sheath 10 together with the first needle catheter 14 have been removed. The thread 30 is fed through the orifice 84 in the fabric 78 of the stentgraft 76. The orifice 84 is located in an area of the fabric 78 that borders on the opening 87 of the branch artery 70 to the aorta 66.

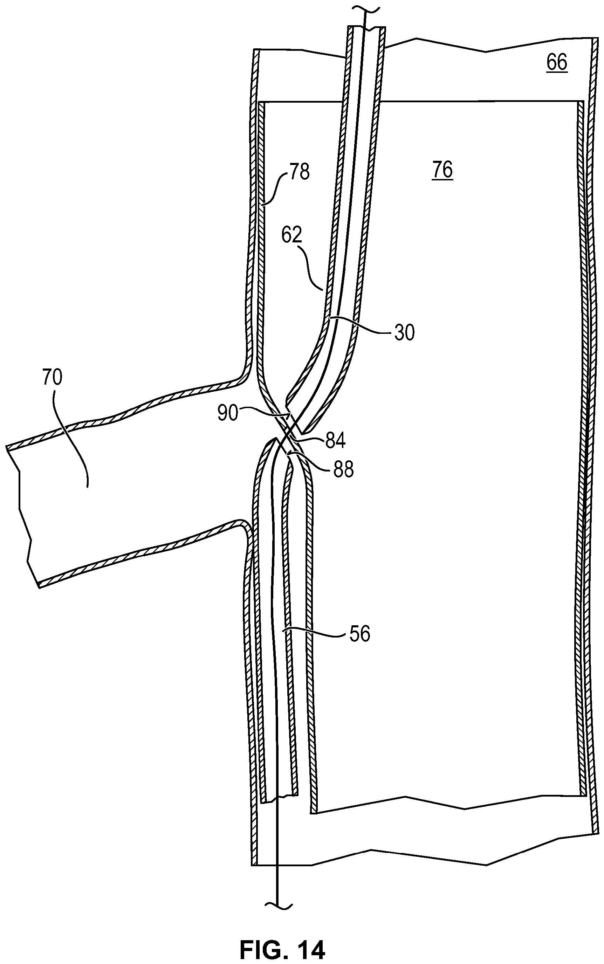

Figure 14 shows a sectional view of the branch region of the branch artery 70 and the aorta 66 with the second introducer sheath 56 and the third introducer sheath 62 introduced into the aorta 66. In order to introduce the introducer sheaths 56 and 62, the nylon thread 30 has been tensioned. Further, the opening 88 of the second introducer sheath 88 faces the fabric 78 in proximity to the orifice 84. The opening 90 of the third introducer sheath 62 faces the opposite inner side of the fabric 78 and is located in close proximity to the orifice 84.

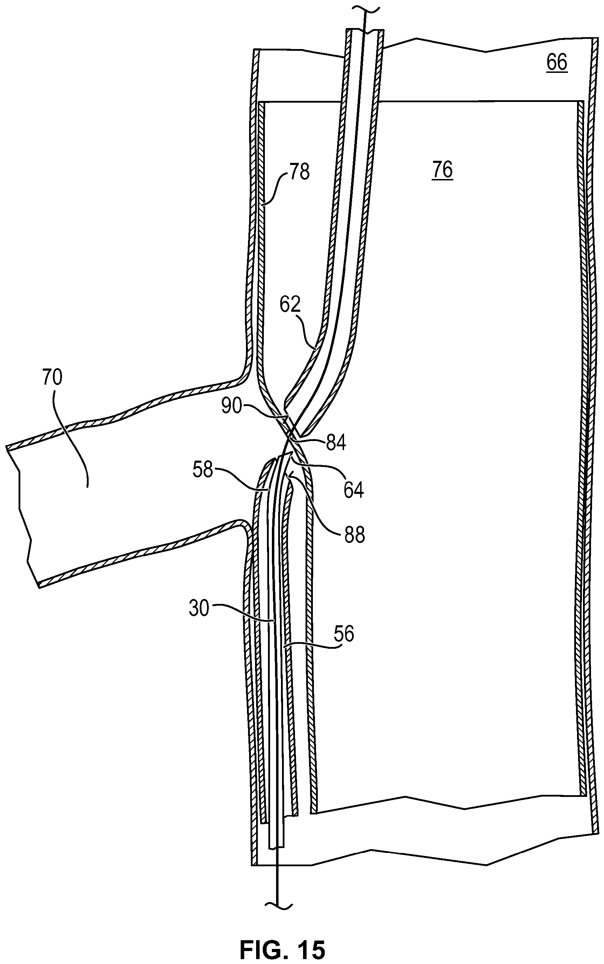

Figure 15 shows a sectional view of the branch region of the branch artery 70 and the aorta 66, wherein the tip of the second needle catheter 58 is moved close to the orifice 84 of the fabric 78. The second needle catheter 58 is introduced in the introducer sheath 56 over the nylon thread 30 put under tension. The tip of the catheter 58 contains the hollow needle 64 acting as a small dilator over the wire 30 lying outside the fabric 78. The tip of the second needle catheter 58 is moved through the fabric 78 in the region of the orifice 84, so that the orifice 84 is enlarged, and further along the thread 30 into the introducer sheath 62, which prevents an accidental puncture of body tissue of the patient.

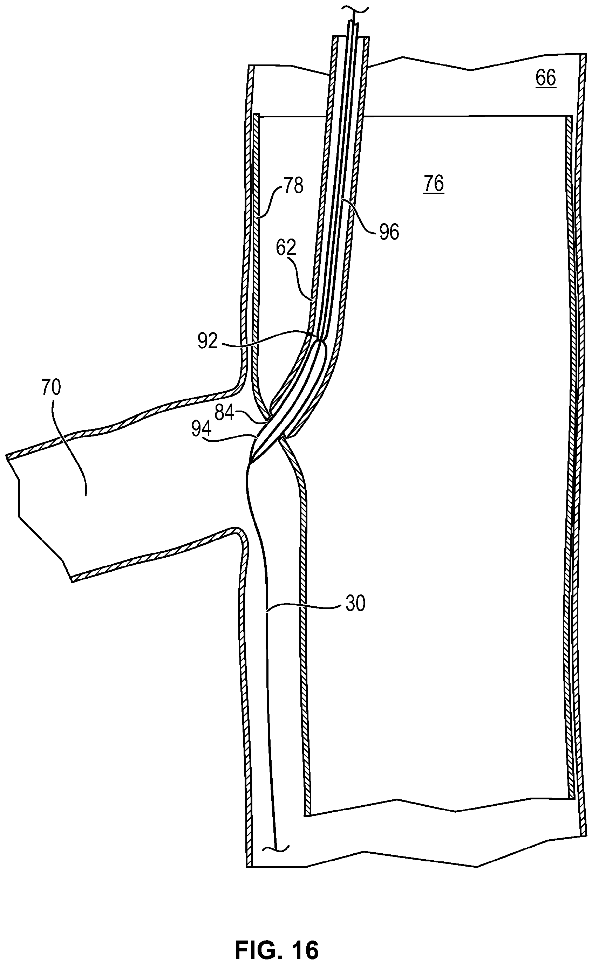

Figure 16 shows a sectional view of the branch region of the branch artery 70 and the aorta 66, wherein the second introducer sheath 56 and the second needle catheter 58 have been removed and an angioplasty balloon catheter 92 has been introduced in an antegrade manner. The angioplasty balloon catheter 92 comprises an angioplasty balloon 94 and a catheter 96.

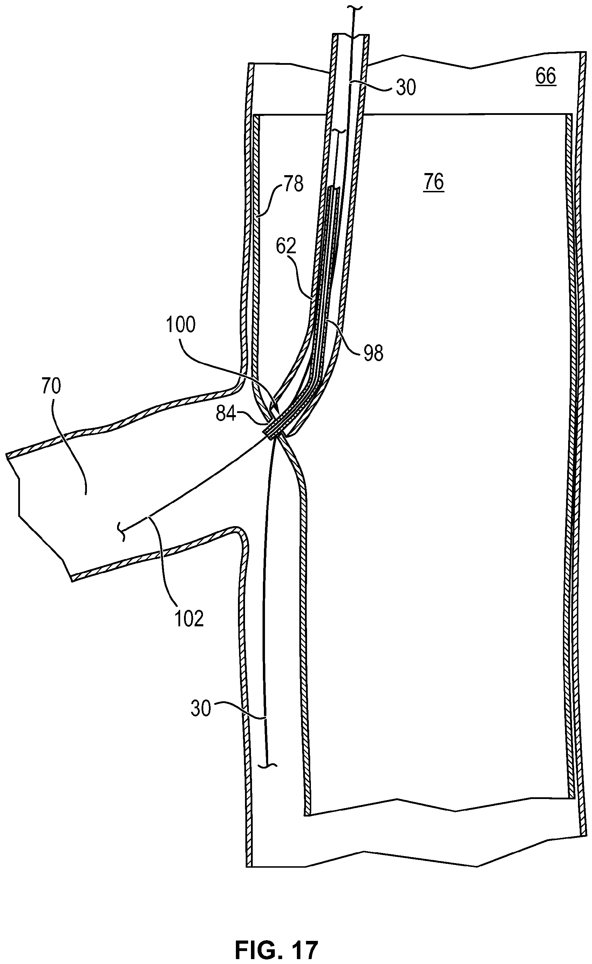

The catheter 96 extends so far into the introducer sheath 62 that the angioplasty balloon 94 penetrates the fabric 78 through its orifice 84 so that the orifice 84 is further enlarged. As a next step, the enlarged orifice 84 is cannulated in an antegrade manner with the help of a diagnostic catheter 98, e.g. a van Schie 3 catheter, as is shown in Figure 17 in a sectional view.

The diagnostic catheter 98 has an angled tip 100 which is moved from the inner side of the fabric 78 through the orifice 84 into the direction of the branch artery 70 after the second needle catheter 58 has been removed from the branch artery 70. With the help of the diagnostic catheter 98 a flexible guide wire 102 is fed through the third introducer sheath 62, the orifice 84 and a part of the branch artery 70.

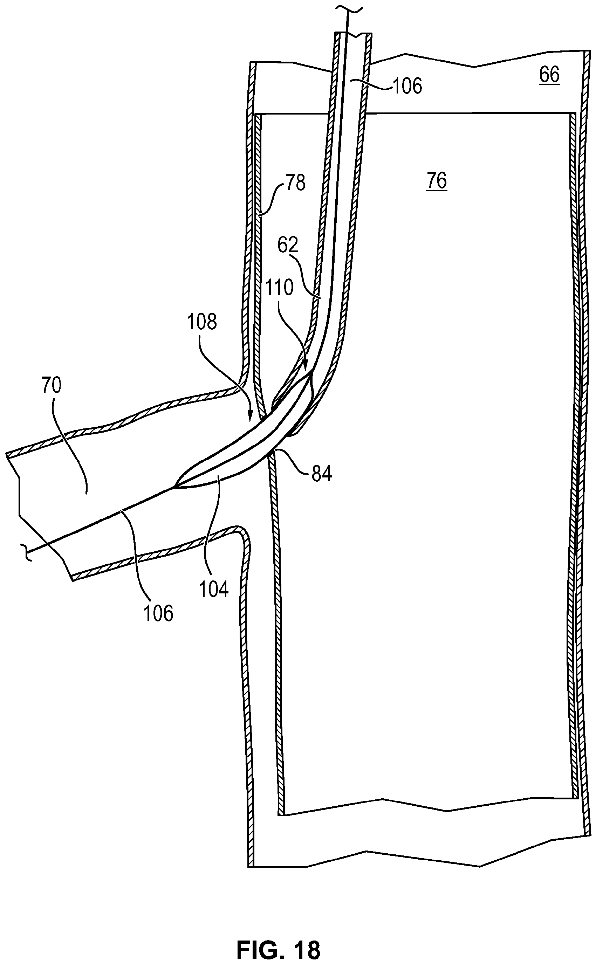

Figure 18 shows a sectional view of the branch region of the branch artery 70 and the aorta 66, wherein a PTA balloon 104 has been introduced through the third introducer sheath 62 after the diagnostic catheter 98 has been removed. In order to introduce the PTA balloon 104, the flexible guide wire 102 has been replaced by a stiff wire 106.

Further, the PTA balloon 104 has been moved along the stiff wire 106 in such a manner through the orifice 84 that a first half 108 is located on the outer side of the fabric 78 and a second half 110 of the PTA balloon 104 is located on the inner side of the fabric 78.

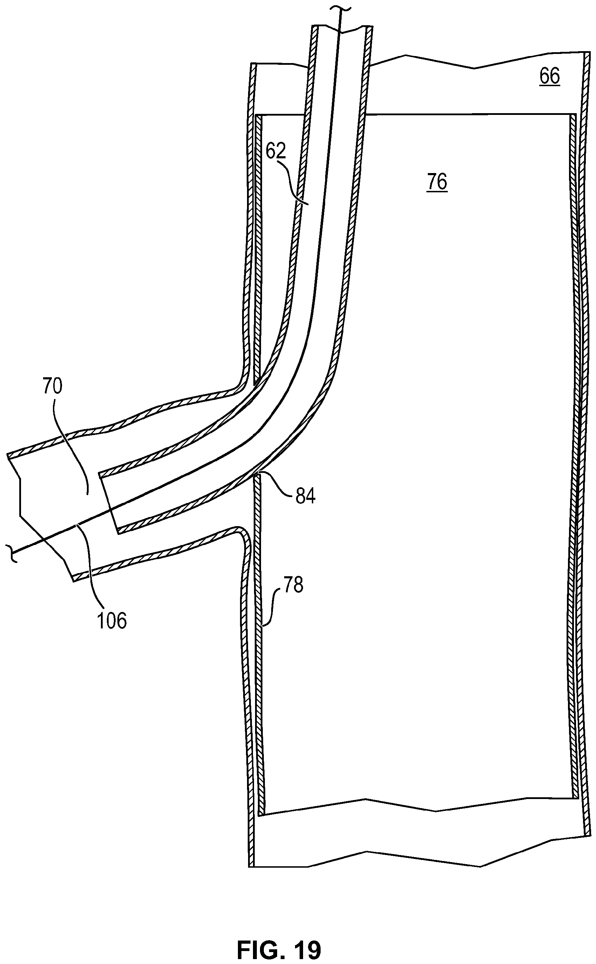

The PTA balloon 104 is subsequently rapidly inflated and deflated several times and at the same time the third introducer sheath 62 is pushed into the direction of the orifice 84 so that the third introducer sheath 62 moves through the fabric 78 along the guide wire 106 into the branch artery 70. The result after the third introducer sheath 62 has entered the branch artery 70 is shown in Figure 19.

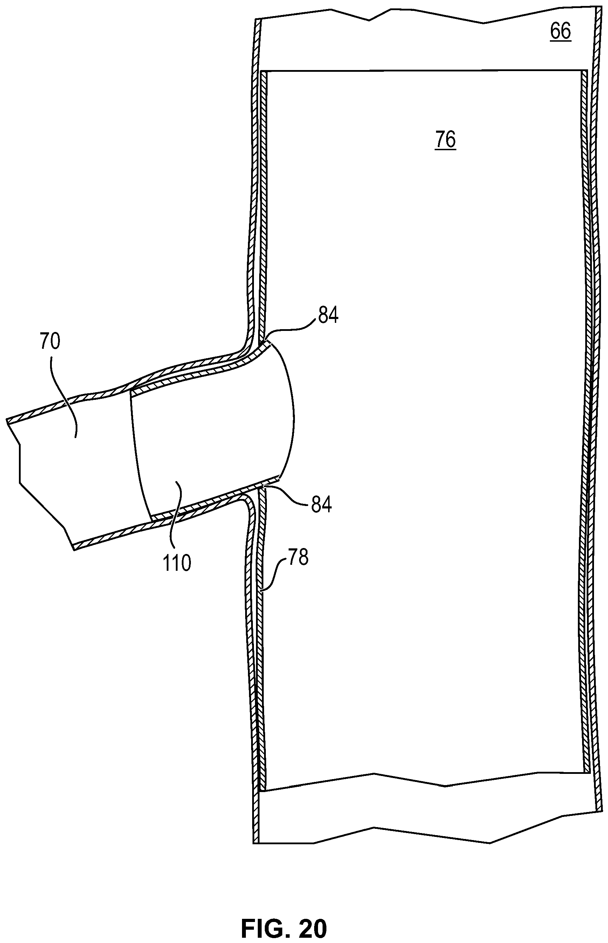

Figure 20 shows a sectional view of the branch region of the branch artery 70 and the aorta 66 after the previous step of the fenestration method in which a sidebranch stent 110 has been placed into the branch artery 70 to seal the stentgraft 76. The side branch stent 110 is fitted through the orifice 84 and lies against the inner walls of the branch artery 70.

### REFERENCES

Berg, P., 2010. Magnetic apparatus for in situ location of a graft fenestration site and method of using same. WO 2009/056644 A1.
Biadillah, Y., Hartley, A., Davies, G., Visram, N., Juzkiw, T., 2012. Stent Graft Fenestration. US 2012/0046657 A1.
Bruszewski, W., Greenan, T., Chu, J., Erickson, D., Morris, J., Ganesan, P., Hanson, C, Rust, M., Thomas, C, 2009a. Fenestration Segment Stent-Graft And Fenestration Method. US2009259290A1.
Bruszewski, W., Rust, M., Greenan, T., 2009b. Device and Method for Stent Graft Fenestration In Situ. WO 2009/064672 A2.
Mafi, M., Bruszewski, W., 2009. Stent Graft Delivery System Including Support For Fenestration in Situ and a Mechanism for Modeling. US 2009/0264988 A1.
Manning, B.J., Ivancev, K., Harris, P.L, 2010. In situ fenestration in the aortic arch. J. Vase. Surg. 52, 491-494.
McLachlan, C.S., Mossop, P., Nixon, I., 2010. Method and Device For Graft Fenestration. US2010106175A1.
McWilliams, R.G., Fearn, S.J., Harris, P.L., Hartley, D., Semmens, J.B., Lawrence-Brown, M.M., 2003. Retrograde fenestration of endoluminal grafts from target vessels: feasibility, technique, and potential usage. J. Endovasc. Ther. 10, 946-952.
McWilliams, R.G., Murphy, M., Hartley, D., Lawrence-Brown, M.M., Harris, P.L, 2004. In situ stent-graft fenestration to preserve the left subclavian artery. J. Endovasc. Ther. 11,170-174.
Mendes, B., Oderich, G.S., Cha, S., Duncan, A.A., Kalra, M., Fleming, M., Gloviczki, P., Macedo, T., Bower, T.C., 2013. Anatomical Feasibility of Off-the-Shelf Fenestrated Stent Grafts to Treat Juxtarenal and Pararenal Abdominal Aortic Aneurysms. J. Vase. Surg. 57, 22S-23S.
Naor, G., Ackerman, H., 2012. Method of Implanting a Stent Graft and Creating a Fenestration therein. US 2012/0239132 A1.
Shaw, E.E., 2012. Stent-Grafts and Devices for in Situ Fenestration of a Stent-Graft at the Site of a Branch Vessel. WO 2012/067823 A3.
Wallace, D.T., Stahler, G.J., 2009. In-Situ Graft Fenestration. 2009/0228020 A1.
Wolf, Y.G., 2012. Stent Graft Fenestration. US 2012/0041544 A1.

### LIST OF REFERENCE NUMERALS

- 10, 56, 62: introducer sheaths
- 12, 80, 102, 106: guide wires
- 14, 58: needle catheters
- 16, 100: tip
- 18, 46, 50, 92, 96, 98: catheters
- 20: bending part
- 22: appliance
- 23, 38: cylinders
- 24, 84: orifices
- 26, 30: threads
- 28, 32, 44, 71, 86, 88, 90: openings
- 33: shiftable element
- 34: half cylinder
- 36: piece of metal
- 37: area
- 40, 64: needles
- 42: barbed hook
- 48, 94, 104: balloons
- 52, 54: layers
- 66: aorta
- 68: aneurysm
- 70, 72, 74: branch arteries
- 76, 110: stentgrafts
- 78: fabric
- 108: halfs
- X: longitudinal axis

## Claims

1. A catheter device for fenestrating a stentgraft (76), comprising:
a first needle catheter (14) with a needle (40) having a barbed hook (42) and a thread (30) which is connected to the needle (40);
**characterized in that** it further comprises a balloon catheter (46) with a balloon (48) that is pierceable by the needle (40) such that the barbed hook (42) prevents a retraction of the needle (40) from the balloon (48).

2. The catheter device according to claim 1, further comprising a second needle catheter (58) with a hollow needle (64) through which the thread (30) is feedable.

3. The catheter device according to claim 2, further comprising an introducer sheath (62) into which the hollow needle (64) is feedable in such a manner that an accidental puncture is prevented.

4. The catheter device according to one of the preceding claims, wherein the first needle catheter (14) comprises an appliance (22) for moving the needle (40) in a specified direction.

5. The catheter device according to claim 4, wherein the first needle catheter (14) has a bendable tip (20), the first needle catheter (14) further comprising a steering catheter (18), and the appliance (22) is connected to a bendable tip of the steering catheter (18) that is located in the bendable tip (20) of the first needle catheter (14).

6. The catheter device according to claim 4 or 5, wherein the appliance (22) comprises an elongated guiding element (23) and a shiftable element (33) that is slidably supported by the guiding element (23) in such a manner that the shiftable element (33) is shiftable along a longitudinal axis of the guiding element (23), and the needle (40) is movable by shifting the shiftable element (33) in the specified direction.

7. The catheter device according to claim 6, wherein the guiding element is a hollow cylinder (23) and the shiftable element (33) is located inside of the hollow cylinder (23).

8. The catheter device according to claim 7, wherein the shiftable element (33) comprises a pressure pin (36) and a further hollow cylinder (38) for supporting the needle (40), and the needle (40) is locatable in such a manner that the needle (40) extends into the hollow further cylinder (38) and that an end of the needle (40) opposite to the tip of the needle (40) abuts against the striker pin (36).

9. The catheter device according to one of the claims 6 to 8, wherein a further thread (26) is connected to the shiftable element (33) in a connection area (37) thereof, and the further thread (26) is fed through an orifice (24) of the guiding element (23) that is located at a distance from the connection area (37) in the specified direction.

10. The catheter device according to one of the claims 5 to 9, wherein the specified direction is a direction opposite to the direction in which the tip (20) of the first needle catheter (14) points.

11. The catheter device according to one of the claims 1 to 7, wherein the balloon (48) has an inner layer (52) and an outer layer (54), and that the inner layer (52) is thicker than the outer layer (54).

## Patentansprüche

1. Kathetervorrichtung zur Fenestrierung eines Stentgrafts (76), umfassend:
einen ersten Nadelkatheter (14) mit einer Nadel (40), die einen Widerhaken (42) aufweist, und einem Faden (30), der mit der Nadel (40) verbunden ist;
**dadurch gekennzeichnet, dass** sie ferner einen Ballonkatheter (46) mit einem Ballon (48) umfasst, der von der Nadel (40) durchbohrbar ist, sodass der Widerhaken (42) eine Retraktion der Nadel (40) aus dem Ballon (48) verhindert.

2. Kathetervorrichtung nach Anspruch 1, ferner umfassend einen zweiten Nadelkatheter (58) mit einer Hohlnadel (64), durch die der Faden (30) durchführbar ist.

3. Kathetervorrichtung nach Anspruch 2, ferner umfassend eine Einführhülle (62), in welche die Hohlnadel (64) derart einführbar ist, dass eine unbeabsichtigte Punktur vermieden wird.

4. Kathetervorrichtung nach einem der vorhergehenden Ansprüche, wobei der erste Nadelkatheter (14) ein Gerät (22) zum Bewegen der Nadel (40) in eine spezifizierte Richtung umfasst.

5. Kathetervorrichtung nach Anspruch 4, wobei der erste Nadelkatheter (14) eine biegbare Spitze (20) aufweist, wobei der erste Nadelkatheter (14) ferner einen Lenkkatheter (18) umfasst und das Gerät (22) mit einer biegbaren Spitze des Lenkkatheters (18) verbunden ist, der sich in der biegbaren Spitze (20) des ersten Nadelkatheters (14) befindet.

6. Kathetervorrichtung nach Anspruch 4 oder 5, wobei das Gerät (22) ein längliches Führungselement (23) und ein verschiebbares Element (33) umfasst, das derart von dem Führungselement (23) verschiebbare gestützt wird, dass das verschiebbare Element (33) entlang einer Längsachse des Führungselements (23) verschiebbar ist und die Nadel (40) durch Verschieben des verschiebbaren Elements (33) in die spezifizierte Richtung verschiebbar ist.

7. Kathetervorrichtung nach Anspruch 6, wobei das Führungselement ein Hohlzylinder (23) ist und sich das verschiebbare Element (33) innerhalb des Hohlzylinders (23) befindet.

8. Kathetervorrichtung nach Anspruch 7, wobei das verschiebbare Element (33) einen Druckstift (36) und einen weiteren Hohlzylinder (38) zum Stützen der Nadel (40) umfasst und die Nadel (40) derart platzierbar ist, dass sich die Nadel (40) in den weiteren Hohlzylinder (38) erstreckt und dass ein Ende der Nadel (40) gegenüber der Spitze der Nadel (40) an den Anschlagsstift (36) angrenzt.

9. Kathetervorrichtung nach einem der Ansprüche 6 bis 8, wobei ein weiterer Faden (26) in einem Verbindungsbereich (37) davon mit dem verschiebbaren Element (33) verbunden ist und der weitere Faden (26) in die spezifizierte Richtung durch eine Öffnung (24) des Führungselements (23) geführt wird, das sich in einem Abstand zu dem Verbindungsbereich (37) befindet.

10. Kathetervorrichtung nach einem der Ansprüche 5 bis 9, wobei die spezifizierte Richtung eine Richtung entgegengesetzt der Richtung ist, in welche die Spitze (20) des ersten Nadelkatheters (14) zeigt.

11. Kathetervorrichtung nach einem der Ansprüche 1 bis 7, wobei der Ballon (48) eine Innenschicht (52) und eine Außenschicht (54) aufweist und wobei die Innenschicht (52) dicker als die Außenschicht (54) ist.

## Revendications

1. Dispositif de cathéter pour la fenestration d'une endoprothèse (76), comprenant :
un premier cathéter-aiguille (14) avec une aiguille (40) ayant un crochet à barbes (42) et un fil (30) qui est relié à l'aiguille (40) ;
**caractérisé en ce qu'**il comprend en outre un cathéter à ballonnet (46) avec un ballonnet (48) qui peut être transpercé par l'aiguille (40) de sorte que le crochet à barbes (42) empêche une rétraction de l'aiguille (40) à partir du ballonnet (48).

2. Dispositif de cathéter selon la revendication 1, comprenant en outre un second cathéter-aiguille (58) avec une aiguille creuse (64) à travers laquelle le fil (30) peut être introduit.

3. Dispositif de cathéter selon la revendication 2, comprenant en outre une gaine d'introduction (62) dans laquelle l'aiguille creuse (64) peut être introduite de manière à empêcher une ponction accidentelle.

4. Dispositif de cathéter selon l'une quelconque des revendications précédentes, dans lequel le premier cathéter-aiguille (14) comprend un accessoire (22) pour déplacer l'aiguille (40) dans une direction spécifiée.

5. Dispositif de cathéter selon la revendication 4, dans lequel le premier cathéter-aiguille (14) possède une pointe pliable (20), le premier cathéter-aiguille (14) comprenant en outre un cathéter de direction (18), et l'accessoire (22) est relié à une pointe pliable du cathéter de direction (18) qui est disposé dans la pointe pliable (20) du premier cathéter-aiguille (14).

6. Dispositif de cathéter selon la revendication 4 ou 5, dans lequel l'accessoire (22) comprend un élément de guidage allongé (23) et un élément déplaçable (33) qui est supporté de manière coulissante par l'élément de guidage (23) de manière à ce que l'élément déplaçable (33) punisse être déplacé le long d'un axe longitudinal de l'élément de guidage (23), et à ce que l'aiguille (40) soit mobile par le déplacement de l'élément déplaçable (33) dans la direction spécifiée.

7. Dispositif de cathéter selon la revendication 6, dans lequel l'élément de guidage est un cylindre creux (23) et l'élément déplaçable (33) est disposé à l'intérieur du cylindre creux (23).

8. Dispositif de cathéter selon la revendication 7, dans lequel l'élément déplaçable (33) comprend une broche de pression (36) et un cylindre creux supplémentaire (38) pour supporter l'aiguille (40), et l'aiguille (40) peut être positionnée de telle manière que l'aiguille (40) s'étend dans le cylindre creux supplémentaire (38) et qu'une extrémité de l'aiguille (40) opposée à la pointe de l'aiguille (40) bute contre la broche de percussion (36).

9. Dispositif de cathéter selon l'une quelconque des revendications 6 à 8, dans lequel un fil supplémentaire (26) est relié à l'élément déplaçable (33) dans une zone de liaison (37) de celui-ci, et le fil supplémentaire (26) est introduit à travers un orifice (24) de l'élément de guidage (23) qui est disposé à une distance de la zone de liaison (37) dans la direction spécifiée.

10. Dispositif de cathéter selon l'une quelconque des revendications 5 à 9, dans lequel la direction spécifiée est une direction opposée à la direction dans laquelle est orientée à pointe (20) du premier cathéter-aiguille (14).

11. Dispositif de cathéter selon l'une quelconque des revendications 1 à 7, dans lequel le ballonnet (48) possède une couche interne (52) et une couche externe (54), et la couche interne (52) est plus épaisse que la couche externe (54).
